Europäisches Patentamt

**European Patent Office**

**Office européen des brevets**

(11) Publication number: **0 232 753**
A2

# EUROPEAN PATENT APPLICATION

(21) Application number: **87100702.7**

(22) Date of filing: **20.01.87**

(51) Int. Cl.⁴: **A 61 K 35/80**

(30) Priority: **24.01.86 JP 13060/86**

(43) Date of publication of application: **19.08.87**
**Bulletin 87/34**

(84) Designated Contracting States: **AT BE CH DE FR GB LI NL SE**

(71) Applicant: **CHLORELLA INDUSTRY CO., LTD, 5th Floor, Toyokuni Building No. 4-6 Shiba Daimon 2-chome, Minato-ku Tokyo (JP)**

(72) Inventor: **Tanaka, Kuniaki, 1575-1, Arakimachiaraki, Kurume-shi Fukuoka-ken (JP)**

(74) Representative: **Strasse, Joachim, Dipl.-Ing. et al, Strasse und Stoffregen European Patent Attorneys Zweibrückenstrasse 17, D-8000 München 2 (DE)**

(54) Therapeutic agent for neutrophil insufficiency.

(57) An extract having a molecular weight of 5,000 or more obtained by extracting from a Chlorella alga in an aqueous solvent has an effect of improving the chemotaxis and bactericidal function of neutrophils. The extract can be used in therapy for patients suffering from neutrophil insufficiency.

- 1 -

Therapeutic agent for neutrophil insufficiency

The present invention relates to a therapeutic agent for neutrophil insufficiency.

Leukocytes are classified into polymorphonuclear leukocytes and monocytes, in accordance with the shape of their nuclei. Polymorphonuclear leukocytes are further classified into neutrophils, eosinophils, and basophils in accordance with staining properties of their intranuclear granules. Neutrophils account for about 50 % of the total number of leukocytes.

When foreign bodies such as bacteria enters a human body, neutrophils perform chemotaxis, phagocytosis, and bactericidal function to digest them and protect the human body from them. Recent studies have demonstrated that neutrophils are also associated with specific immunological responses. If immunological responses are abnormal, a human body is usually easily infected with various kinds of bacteria (in particular, E. Coli and P. aerginosa). Typical diseases resulting from deficient immunological responses include chronic granulomatosis, Chediac-Higashi syndrome, Hodgkin's disease, and the like. It is well known that premature newborn frequently have an insufficient neutrophil function. In view of this, development of a therapeutic agent for neutrophil insufficiency, i.e., a therapeutic agent for improving deficiency in neutrophil function has been greatly desired. However, no such agent has been produced to date.

It is an object of the present invention to provide a therapeutic agent for neutrophil insufficiency, which contains as an effective component a substance having a molecular weight of 5,000 or more in an extract from an alga of the genus Chlorella in an aqueous solvent.

The effective component of this agent is obtained by extracting from a selected alga in an aqueous solvent at 4 to 98°C for 15 minutes to 1 hour, but it is preferable to extract the effective component in water at 93 to 98°C for 15 to 40 minutes. The extract may or may not by purified by gel filtration chromatography or dialysis with water.

It was found that this extract enhances the chemotaxis and bactericidal function of neutrophils and improves neutrophil insufficiency which results in the various diseases described above.

A therapeutic agent for neutrophil insufficiency according to the present invention is prepared by the following method.

Chlorella as a starting material is prepared. Any alga of the genus Chlorella can be used including C. bulgaris, C. regularis and C. eripsoidia. Methods of culturing algae of the genus Chlorella are well known. More specifically, a chlorella alga is cultured by heteroculture requiring sunlight, carbon dioxide gas and various nutrients. For example, a chlorella alga can be cultured under stirring in an outdoor pool containing acetic acid or glucose as a carbon source, urea or the like as a nitrogen source, and potassium phosphate as a potassium/phosphorus source.

The cultured chlorella alga is then subjected to extraction by aqueous solvent. Prior to extraction, the alga is preferably spray-dried. For example, the alga can be dried by blowing it with hot air at 120°C or higher and dehydrated. The solvent to be used therefor may include water, acid solutions (e.g.,

0.2N hydrochloric acid) and base solutions (e.g., 0.2N sodium hydroxide) having a pH of 5 to 9, and water-containing lower alcohols (e.g., methanol or ethanol). Although the spray-dried alga can be subjected to extraction by an aqueous solvent like the above at 4 to 98°C for 15 minutes to 1 hour, it is preferably subjected to extraction by water at 93 to 98°C for 15 to 40 minutes. The ratio of the alga to the aqueous solvent can be, for exammple, 1 g of alga to 20 ml of solvent. The objective chlorella extract (hereinafter referred to as CE) is obtained by extracting from the alga, centrifuging the solvent extract, and recovering the supernatant.

The resultant CE can be used directly as a therapeutic agent for neutrophil insufficiency. However, in order to improve the effect of the agent, a fraction having a molecular weight of 5,000 or more (hereinafter referred to as CVE-A) is preferably recovered from the CE through purification. The fraction having a molecular weight of 5,000 or more can be obtained through gel filtration chromatography or by dialysis against water. When the fraction is obtained by gel filtration chromatography, "Sephadex G-25" (trade name) can be used as the gel-filtering substance. The CVE-A is obtained through such purification process. Although unproven, the CVE-A obtained in the one of the above purification processes is assumed to consist of many types of polysaccharides and proteins.

The CE or CVE-A obtained in the above manner can be used as a therapeutic agent for neutrophil insufficiency, and need not be subjected to any special treatment prior to use as such. However, for easy administration and storage, the CE or CVE-A is preferably freeze-dried.

When the CE or CVE-A is administered to a human subject, it can be administered orally, subcutaneously or intravenously. The amount normally required for an adult subject is normally 500 to 2,000 mg/day when given orally and 50 to 200 mg/day when given subcutaneously or intravenously.

When the CE or CVE-A is provided as a therapeutic drug for neutrophil insufficiency, a pellet or powder containing 50 g of CVE-A, 30 g of lactose and 20 g of dextran can be prepared for oral administration. For subcutaneous or intravenous administration, a solution of the CVE-A in a physiological saline solution in a concentration of 20 mg/mℓ is prepared, and 5 mℓ are charged in an ampoule.

The $LD_{50}$ of the CVE-A for mice is 4 g/kg or more for oral administration and 500 mg/kg or more for subcutaneous or intravenous administration.

The effect of the CE or CVE-A in restoring neutrophil function is evaluated by measuring the bactericidal function of neutrophils, i.e., measuring the amount of superoxide produced thereby. This effect can also be evaluated by measuring the chemotaxis of neutrophils. Accordingly, the CE or CVE-A can also be used as an agent for testing neutrophil function.

Example 1

Preparation of Therapeutic Agent for Neutrophil Insufficiency

Chlorella vulgaris was cultured by conventional heteroculture. The culture was performed under stirring in an outdoor pool containing acetic acid or glucose as a carbon source, urea or the like as a nitrogen source, and potassium phosphate as a potassium/phosphorus source. The recovered chlorella alga was blow-dried at 120°C. One gram of the dried alga was placed in 20 mℓ of water at 93°C, and the water was heated at 93 to 98°C for 15 minutes under frequent stirring. Thereafter, the water containing the alga was cooled to 4°C and cen-trifuged at 6,000 rpm for 15 minutes. The supernatant was recovered as the CE. The CE was gel-filtered with "Sephadex G-25" (trade name) to obtain a fraction (CVE-A) having a molecular weight of 5,000 or more.

Example 2

Reinforcement of Superoxide Productivity of
Neutrophils by CVE-A

CVE-A was administered to mice at a dosage of
50 mg/kg. Control mice were administered a physiological saline solution. After 24 hours, 2 mℓ of a
2 % casein solution were abdominally administered to
each mouse. This was administered to concentrate
neutrophils in the abdominal cavity. After 4 more
hours, a peritoneal exudation cel (PEC) solution containing a large number of neutrophils was sampled from
the abdominal cavity of each mouse. The sampled PEC
solution was centrifuged at 110 × g or 10 minutes, and
the centrifuged cells were stained with tripan blue.
The number of live cells was then counted (deal cells
were stained).

In Experiment 1, 10 mice were administered the
CVE-A, and the PEC solutions obtained from the each
mouse were mixed together. The PEC concentration in
this mixture was adjusted to $5 \times 10^6$ cells/mℓ with
a phosphate buffered saline. In Experiment 2, 10 mice
were administered the CVE-A, and the PEC solutions
from two mice were collected to prepare 5 samples.
The PEC concentration in each sample was adjusted to
$3 \times 10^6$ cells/mℓ with the same phosphate buffered
saline.

The superoxide productivity of each sample was measured in the following manner: 0.1 mℓ of a PEC suspension, 0.02 mℓ Of 5 mM cytochrome solution, and 0.85 mℓ
of a reaction solution having a pH of 7.4 (17 mM hepes,
120 mM salt, 2 mM magnesium sulfate, 0.5 mM calcium
chloride, and 5 mM glucose) were charged into a plastic
absorbancy measurement cell. The cell was allowed to
stand still at 37°C for 10 minutes. Subsequently, 2μℓ
of 1 mg/mℓ phorbol myristate acetate were quickly added
and stirred. The cell was set on a two-wavelength spectroscope (Model 556 available from Hitachi, Ltd.) and an absorbancy difference between 550 nm and 540 nm after standing at 37°C for 10 minutes was determined. Since the absorbancy difference corresponds to the amount of cytochrome C reduced by the superoxide, it can be calculated in terms of the amount of superoxide produced. The obtained results are shown in Table 1 below. The values are average values of five measurements $\pm$ standard errors.

Table 1

| CVE-A | Neutrophil Conten in PEC (%) | Amount of Produced Superoxide $(nmol\ O_2-/min\cdot 10^5\ neutrophils)$ |
|---|---|---|
| Experiment 1 | | |
| − | 70 | $0.406 \pm 0.024$ |
| + | 70 | $0.642 \pm 0.030$ |
| Experiment 2 | | |
| − | 55 | $0.548 \pm 0.217\cdot$ |
| + | 59 | $0.927 \pm 0.131$ |

As can be seen from Table 1, the amount of superoxide produced by the PEC in the group treated with the CVE-A is about 1.6 times that of the control group. This demonstrates the fact that the CVE-A has an effect of improving the bactericidal function of neutrophils.

Example 3

Improvement in Superoxide Productivity of Deficient Neutrophils by CVE-A

Mouse group A was infected with P. aerginosa in an amount close to a lethal dose, mouse group B was fed a low-protein feed for 2 weeks, and normal mouse group C was prepared. Each group consisted of 10 mice. Each mouse of each group was subcutaneously administered the CVE-A in an amount of 50 mg/kg. As a control, another 10 mice of each group was administered a physiological saline solution.  24 hours after the treatment, the PEC solution was sampled from mice of each group following the procedures of Example 2.  The amounts of superoxide produced were measured following the same procedures as in Example 2.  The obtained results are shown in Table 2.

<u>Table 2</u>

| CVE-A | | Amount of Produced Superoxide ($nmol\ O_2^-/min \cdot 10^5$ neutrophils) |
|---|---|---|
| Group A | - | $0.238 \pm 0.101$ |
| | + | $0.503 \pm 0.087$ |
| Group B | - | $0.154 \pm 0.037$ |
| | + | $0.252 \pm 0.124$ |
| Group C | - | $0.502 \pm 0.026$ |
| | + | $0.886 \pm 0.045$ |

As can be seen from Table 2, by treatment with the CVE-A, neutrophils of not only normal mice but also those of mice having insufficient superoxide productivity due to infection with bacteria or malnutrition have improved functions.

Example 4

<u>Reinforcement of Chemotaxis by CVE-A</u>

The reinforcement effect of the CVE-A was examinedusing the PEC solution samples from a subject's body.  Two mℓ of 2 % casein were administered to a non-treated mouse.  After 4 hours, the PEC solution containing neutrophils was sampled from the abdominal cavity of the mouse.  The PEC concentration was adjusted to $5 \times 10^5$ cells/mℓ using RPMI 1640 solution added 0.5 % fetal bovive serum.  0.2 mℓ of the PEC solution were charged into an upper chamber of each of three chemotaxis measurement chambers (blind chambers available from Nucleopore Inc., Pleasanton, California, U.S.A.) in which respecrive upper and lower chambers are partitioned with a membrane having a number of 5 μm pores. Different concentrations of CVE-A were charged into the lower chambers of the three measurement chambers. Incubation was performed in a 5 % carbon dioxide atmosphere at 37°C for 90 minutes. The membranes between the upper and lower chambers were then removed, and the number of cells transmigrated from the upper to the lower chamber were counted using a microscope. The obtained results are shown in Table 3.

Table 3

| CVE-A Concentration in Lower Chamber ($\mu$g/m$\ell$) | 0 | 10 | 100 |
|---|---|---|---|
| Number of Transmigrated Cells | 1.7 | 8.7 | 22.0 |

As can be seen from Table 3, the higher the CVE-A concentration, the larger the number of neutrophils transmigrating to the lower chamber. This indicates that the CVE-A can improve the mobility of neutrophils.

Example 5

Improvement of Human Neutrophils by CVE-A

Insufficient neutrophil function is observed not only in people suffering from neutrophil function insufficiency, but also in people with seriously bacterial infections or suffering from malnutrition. An experiment was performed using neutrophils from such people. First, peripheral blood of patients A, B and C and that of a normal person was sampled, and neutrophils were separated by a specific gravity separation method. The reinforcement effect of superoxide productivity and chemotaxis of neutrophils by the CVE-A was examined in vitro following the procedures of Examples 2 and 4. The neutrophil concentration was adjusted to $3 \times 10^6$ cells/m$\ell$ using an RPMI 1640 culture medium, and to a final CVE-A

concentration of 10 µg/mℓ. The cell suspension was incubated a 5 % $CO_2$ atmosphere at 37°C for 1 hour, and the amount of superoxide produced by the neutrophils was measured in accordance with the procedures of Example 2. The CVE-A concentration in the lower chamber of the blind chamber was set at 10 µg/mℓ and chemotaxis was evaluated following the same procedures as in Example 4. The obtained results are shown in Table 4.

Table 4

| CVE-A | | Amount of Produced Superoxide (nmol $O_2-$/min·$10^5$ neutrophils) | No. of Transmigrated Cells |
|---|---|---|---|
| Healthy Person | − | 5.5 | 3.2 |
| | + | 9.1 | 35.4 |
| Patient A | − | 2.5 | 1.5 |
| | + | 4.0 | 32.9 |
| Patient B | − | 0.9 | 2.1 |
| | + | 1.2 | 3.2 |
| Patient C | − | 1.5 | 0.9 |
| | + | 2.8 | 19.7 |

It can be seen from Table 4 that when neutrophil insufficiency of the sample from patient B was not improved through CVE-A treatment, i.e., when such neutrophil insufficiency is attributed to a genetic factor, improvement in neutrophil function by administration of the CVE-A to pattern B cannot be expected. However, if neutrophil function (superoxide productivity and chemotaxis) is improved by CVE-A treatment in vitro as in the samples from patients A and C, an improvement in neutrophil function by administration of a substance having the improvement function such as CVE-A can be expected in vivo.

Claims:

1. A therapeutic agent for neutrophil insufficiency, comprising as an effective component a substance having a molecular weight of not less than 5,000 in an extract extracted from an alga of the genus Chlorella by an aqueous solvent.

2. An agent according to claim 1, characterized in that the substance is obtained by extraction in the aqueous solvent at 4 to 98°C for 15 minutes to 1 hour.

3. An agent according to claim 2, characterized in that the aqueous solvent is water, and extraction is performed at 93 to 98°C for 15 minutes to 40 minutes.

4. A therapeutic agent for neutrophil insufficiency, comprising as an effective component a substance which has a molecular weight of not less than 5,000 and is obtained by extracting from an alga of the genus Chlorella by an aqueous solvent and purifying the extract by gel filtration chromatography or dialysis against water.